# EUROPEAN PATENT APPLICATION

(11) **EP 1 640 364 A1**
(43) Date of publication of application: **29.03.2006**
(21) Application number: 04746215.5
(22) Date of filing: 22.06.2004
(51) Int. Cl.: C07D 207/14, C07D 207/12, C07D 241/04, B01J 23/44, C07B 61/00

(54) **PROCESS FOR PRODUCING NITROGENOUS HETEROCYCLIC COMPOUND**

(30) Priority: 27.06.2003 JP 2003184057; 27.06.2003 JP 2003184058; 24.12.2003 JP 2003426663
(71) Applicant: Toray Fine Chemicals Co., Ltd., Urayasu-shi, Chiba 279-8555 (JP)
(72) Inventor: MORII, Seiji, 4610001 (JP); ONO, Takae, 4770032 (JP); SATO, Haruyo, 4540926 (JP)
(74) Representative: Coleiro, Raymond
(86) International application number: PCT/JP2004/008746
(87) International publication number: WO 2005/000810

(57) **Abstract**

A nitrogenous heterocyclic compound such as 3-aminopyrrolidine derivative is produced by hydrogenolysis of an N-substituted nitrogenous heterocyclic compound with normal pressure hydrogen in a water-based solvent in presence of a catalyst. In the case an optically active 1-substituted-3-aminopyrrrolidine derivative is used as a raw material, an optically active 3-aminopyrrolidine derivative can be obtained as a product practically without racemination.

## Description

### Technical Field

The invention relates to a process for producing a nitrogenous heterocyclic compound which is an important compound and a process for producing a nitrogenous heterocyclic compound having a protected amino group in the side chain and also an optically active nitrogenous heterocyclic compound having a protected amino group, as a raw material for synthesis of pharmaceuticals and agricultural agents.

### Background Art

Conventionally, as a method for removing benzyl group which is a protection group for N atom of a nitrogenous heterocyclic compound by hydrogenolysis has been known a method of carrying out hydrogenolysis under hydrogen pressure, however use of the pressurized hydrogen not only requires a pressure resistant facility but also is problematic in terms of the safety. Practically, a method for producing 3-(tertiarybutoxycarbonylamino)pyrrolidine (hereinafter tertiary is abbreviated as t) by hydrogenolysis of 1-benzyl-3-(t-butoxycarbonylamino)pyrrolidine under hydrogen pressure of several kg/cm² has been known well (e. g. , reference to Patent Document No. 1).
Patent Document No. 1: Japanese Patent No. 2,995,704.

### DISCLOSURE OF THE INVENTION

### Problems to be Solved by the Invention

The aim of the invention is to provide an industrially advantageous method for removing a benzyl group, which is a protection group for N atom of a nitrogenous heterocyclic compound, in consideration of safety by using a widely employed facility.

### Means for Solving the Problems

According to the invention to accomplish the above-mentioned aim, it is made possible to carry out debenzylation of a benzyl group, which is a protection group for N atom of a nitrogenous heterocyclic compound by hydrogenolysis with normal pressure hydrogen. That is, an N-substituted nitrogenous heterocyclic compound defined by the general formula (1): 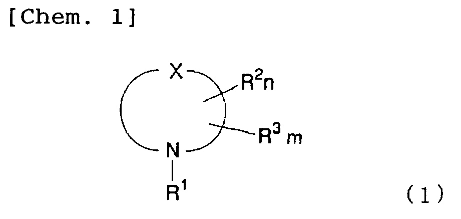 wherein R¹ denotes an (un)substituted benzyl group; R² and R³ independently denote i) a hydrogen atom; ii) a lower alkyl having 1 to 4 carbon atoms; iii) a lower alkoxyl having 1 to 4 carbon atoms; iv) a hydroxyl group; v) a mercapto group; vi) an (un) substituted amino group; vii) an aryl whose aromatic ring may be unsubstituted or substituted with an alkyl having 1 to 4 carbon atoms, an alkoxyl having 1 to 4 carbon atoms, or a halogen atom; or viii) an aralkyl whose aromatic ring may be unsubstituted or substituted with an alkyl having 1 to 4 carbon atoms, an alkoxyl having 1 to 4 carbon atoms, or a halogen atom and may be the same or different groups; m and n independently denote an integer of 0 to 3; and X denotes a residual group of a nitrogenous heterocyclic ring defined by the general formula (2): 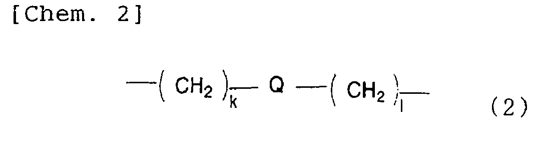 wherein Q denotes CH₂, NR⁴, or O, wherein R⁴ denotes a hydrogen atom; ii) a lower alkyl having 1 to 4 carbon atoms; iii) a lower alkoxyl having 1 to 4 carbon atoms; iv) an aryl whose aromatic ring may be unsubstituted or substituted with an alkyl having 1 to 4 carbon atoms, an alkoxyl having 1 to 4 carbon atoms, or a halogen atom; v) an aralkyl whose aromatic ring may be unsubstituted or substituted with an alkyl having 1 to 4 carbon atoms, an alkoxyl having 1 to 4 carbon atoms, or a halogen atom; or vi) an aralkyloxy whose aromatic ring may be unsubstituted or substituted with an alkyl having 1 to 4 carbon atoms, an alkoxyl having 1 to 4 carbon atoms, or a halogen atom; k and 1 independently denote an integer of 1 to 4; and k + 1 is 3 to 6: is a nitrogenous heterocyclic compound having 4 to 7 ring members and may include compounds of which at least one among the atoms composing heterorings is N atom and compounds of which atoms composing the heterorings contain N atom and O atom at least one each.

Hydrogenolysis of the compound defined by the general formula (1) gives a compound defined by the general formula (3) : 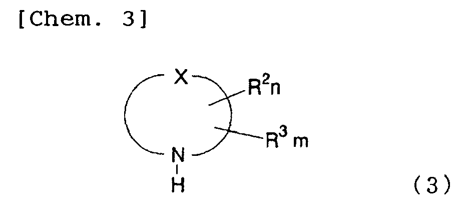 wherein R², R³, X, m, and n independently denote as described above. The invention provides the process for producing the nitrogenous heterocyclic compound defined by the general formula (3). A Pd/C catalyst is used in a water-based solvent for reaction in a preferable embodiment of the invention. The reason for that the reaction is not carried out in a water-based solvent is because it is expected that the dispersibility of the Pd catalyst is inhibited to delay the promotion of reaction due to toluene produced in hydrogenolysis or separation of substituted toluene layers from water. Further, it is expected that in the case the nitrogenous heterocyclic compound defined by the general formula (3), which is a product, is dissolved in water, recovery of the compound from the reaction solution is complicated and it becomes difficult to obtain a pure product with a low water content. However, surprisingly, according to the invention, it is found that hydrogenolysis is carried out in a water-based solvent and the debenzylation speed is rather improved and therefore the aim can be accomplished in co-presence of normal pressure hydrogen without requiring any special pressure-resistant reaction apparatus and finally the invention has been completed.

### Effects of the Invention

According to the invention, using a conventionally widely used facility, the benzyl group, a protection group for the N atom of the nitrogenous heterocyclic compound, can be removed by an industrially advantageous method in consideration of safety. Further, if an optically active isomer is used as a raw material, debenzylation is carried out to produce the optically active nitrogenous heterocyclic compound without deteriorating the optical purity.

### Best Mode for carrying out the Invention

Hereinafter, the best mode of embodiments of the invention will be described. That is, an N-substituted nitrogenous heterocyclic compound defined by the general formula (1): 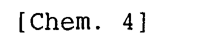 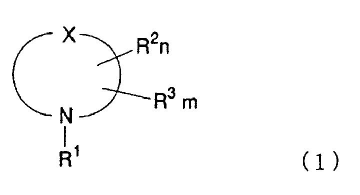 wherein R¹ denotes an (un)substituted benzyl group; R² and R³ independently denote i) a hydrogen atom; ii) a lower alkyl having 1 to 4 carbon atoms; iii) a lower alkoxyl having 1 to 4 carbon atoms; iv) a hydroxyl group; v) a mercapto group; vi) an (un) substituted amino group; vii) an aryl whose aromatic ring may be unsubstituted or substituted with an alkyl having 1 to 4 carbon atoms, an alkoxyl having 1 to 4 carbon atoms, or a halogen atom; or viii) an aralkyl whose aromatic ring may be unsubstituted or substituted with an alkyl having 1 to 4 carbon atoms, an alkoxyl having 1 to 4 carbon atoms, or a halogen atom and may be the same or different groups; m and n independently denote an integer of 0 to 3; and X denotes a residual group of a nitrogenous heterocyclic ring defined by the general formula (2) : 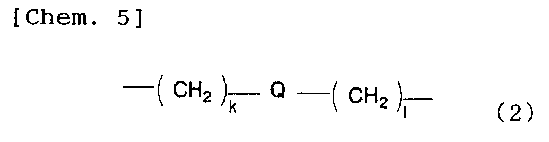 wherein Q denotes CH₂, NR⁴, or O, wherein R⁴ denotes a hydrogen atom; ii) a lower alkyl having 1 to 4 carbon atoms; iii) a lower alkoxyl having 1 to 4 carbon atoms; iv) an aryl whose aromatic ring may be unsubstituted or substituted with an alkyl having 1 to 4 carbon atoms, an alkoxyl having 1 to 4 carbon atoms, or a halogen atom; v) an aralkyl whose aromatic ring may be unsubstituted or substituted with an alkyl having 1 to 4 carbon atoms, an alkoxyl having 1 to 4 carbon atoms, or a halogen atom; or vi) an aralkyloxy whose aromatic ring may be unsubstituted or substituted with an alkyl having 1 to 4 carbon atoms, an alkoxyl having 1 to 4 carbon atoms, or a halogen atom; k and l independently denote an integer of 1 to 4; and k + l is 3 to 6: is a nitrogenous heterocyclic compound having 4 to 7 ring members and may include compounds of which at least one among the atoms composing heterorings is N atom and compounds of which atoms composing the heterorings contain N atom and O atom at least one each.

Hydrogenolysis of the compound defined by the general formula (1) gives a compound defined by the general formula (3) : 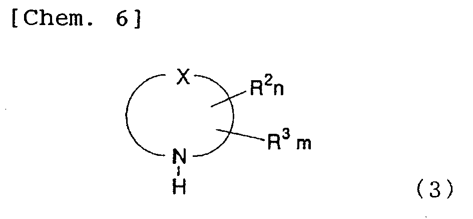 wherein R², R³, X, m, and n independently denote as described above.
Practical examples of the N-substituted nitrogenous compound defined by the general formula (1) are N-benzylpyrrolidine derivatives such as 3-amino-1-benzylpyrrolidine, 3-amino-1-(4-methylbenzyl)pyrrolidine, 3-methylamino-l-benzylpyrrolidine, 1-benzyl-3-tert-butoxycarbonylaminopyrrolidine, 3-benzylamino-1-benzylpyrrolidine, 1-benzyl-3-hydroxypyrrolidine, 1-benzyl-3-methoxypyrrolidine, 3-amino-1-benzyl-4-hydroxypyrrolidine, 3-benzylamino-1-benzyl-4-hydroxypyrrolidine, 2-aminomethyl-1-benzylpyrrolidine, 2-hydroxymethyl-1-benzylpyrrolidine, and 3-ethoxycarbonylamino-1-(4-methylbenzyl)pyrrolidine; N-benzylpiperidine derivatives such as 3-amino-1-benzylpiperidine and 1-benzyl-3-methylpiperidine; N-benzylhexamethyleneimine derivatives such as 3-amino-1-benzylhexamethyleneimine; N-benzylpiperazine derivatives such as 1-benzyl-3-methylpiperazine and 1,4-dibenzyl-3-methylpiperazine; N-benzyloxazoline derivatives such as 4,4-dimethyl-3-benzyloxazoline; and N-benzylmorpholine derivatives such as 2,6-dimethyl-1-benzylmorpholine and preferable examples are N-substituted nitrogenous heterocyclic compounds defined by the general formulas (4) to (7): 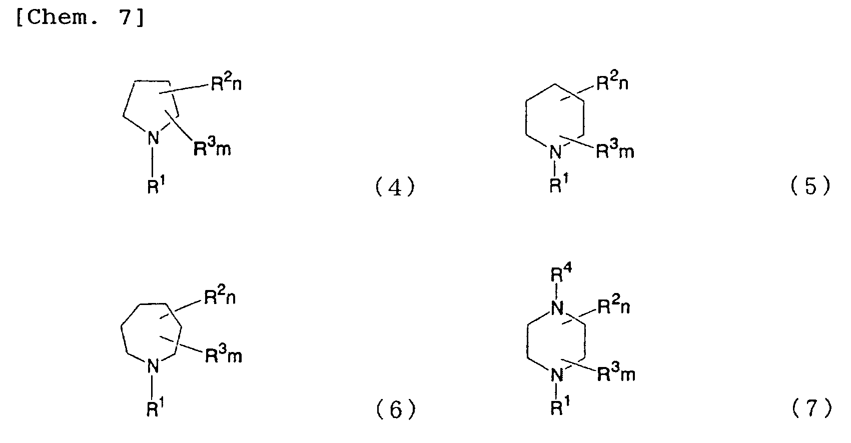 wherein R¹, R², R³, R⁴, m, and n independently denote as described above. Hydrogenolysis of the compounds defined by the general formulas (4) to (7) give compounds defined by the following general formulas (8) to (11): 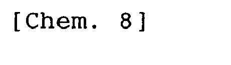 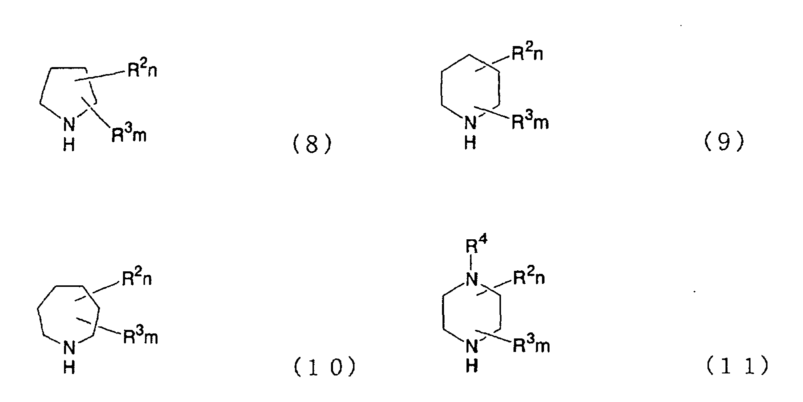 wherein R², R³, R⁴, m, and n independently denote as described above. R² R³, and R⁴ in the general formulas (4) to (11) are preferably a hydrogen atom, methyl, aminomethyl, hydroxymethyl, ethyl, hydroxyl, amino, methylamino, benzylamino, ethoxycarbonylamino, tert-butoxycarbonylamino, benzyloxycarbonylamino, methoxy, and benzyl.

Practical examples of the N-substituted nitrogenous compounds defined by the general formulas (4) to (7) may include N-benzylpyrrolidine derivatives such as 3-amino-1-benzylpyrrolidine, 3-amino-1-(4-methylbenzyl)pyrrolidine, 3-methylamino-1-benzylpyrrolidine, 1-benzyl-3-tert-butoxycarbonylaminopyrrolidine, 3-benzylamino-1-benzylpyrrolidine, 1-benzyl-3-hydroxypyrrolidine, 1-benzyl-3-methoxypyrrolidine, 3-amino-1-benzyl-4-hydroxypyrrolidine, 3-benzylamino-1-benzyl-4-hydroxypyrrolidine, 2-aminomethyl-1-benzylpyrrolidine, 2-hydroxymethyl-1-benzylpyrrolidine, and 3-ethoxycarbonylamino-1-(4-methylbenzyl)pyrrolidine; N-benzylpiperidine derivatives such as 3-amino-1-benzylpiperidine and 1-benzyl-3-methylpiperidine; N-benzylhexamethyleneimine derivatives such as 3-amino-1-benzylhexamethyleneimine; and N-benzylpiperazine derivatives such as 1-benzyl-3-methylpiperazine and 1,4-dibenzyl-3-methylpiperazine: and particularly preferable examples are 3-aminol-benzylpyrrolidine, 1-benzyl-3-tert-butoxycarbonylaminopyrrolidine, 1-benzyl-3-hydroxypyrrolidine, 3-amino-1-benzyl-4-hydroxypyrrolidine, 3-benzylamino-1-benzyl-4-hydroxypyrrolidine, 3-amino-1-benzylpiperidine, and 1-benzyl-3-methylpiperazine.

These N-substituted nitrogenous heterocyclic compounds may include optically active compounds. Use of the optically active compounds of the nitrogenous heterocyclic compounds defined by the general formulas (1) or (4) to (7) makes it possible to produce optically active compounds of the nitrogenous heterocyclic compounds defined by the general formulas (3) and (8) to (11) with scarcely causing racemination. Practically, optically active 3-amino-1-benzylpyrrolidine, optically active 1-benzyl-3-tert-butoxycarbonylaminopyrrolidine, optically active 1-benzyl-3-hydroxypyrrolidine, optically active 3-amino-1-benzyl-4-hydroxypyrrolidine, optically active 3-benzylamino-1-benzyl-4-hydroxypyrrolidine, optically active 3-amino-1-benzylpiperidine, and optically active 1-benzyl-3-methylpiperazine may be used preferably. Also are usable optically compounds having two substituent groups in the nitrogenous heterocyclic ring such as 3-amino-1-benzyl-4-hydroxypyrrolidine. Here, the optically active compounds mean compounds containing 90% or more of either (S) isomers or (R) isomer.

The process for producing the nitrogenous heterocyclic compound of the invention is preferable to be carried out in a water-based solvent. If the solvent contains mainly water, the process can be carried out without a problem even if the solvent contains an organic solvent, however generally the ratio of water in the solvent is 50% or higher, preferably 80% or higher, and more preferably 90% or higher. Examples of the organic solvent allowed to coexist are practically alcohols such as methanol, ethanol, tert-butanol, and benzyl alcohol; and toluene and substituted toluene. They are reaction solvents in the case of synthesis of the N-substituted nitrogenous heterocyclic compound or substances produced in hydrogenolysis. In the case the N-substituted nitrogenous heterocyclic compound is not dissolved in water at all, the reaction is not promoted or is promoted very slowly. In such a case, a slight amount of an acid is added to the reaction solution to convert a portion to be an acid salt and accordingly increase the water-solubility for promoting the reaction.

Also, a method of adding a slight amount of an organic solvent and bringing the solvent into contact with the catalyst may be employed.

If the substrate concentration in the reaction solution is proper to make stirring possible, the reaction can be carried out, and it is generally 1 to 50% by weight, preferably 5 to 40% by weight, and particularly preferably 10 to 30% by weight. If it is within the range, the workability becomes excellent and the production yield is high.

As the catalyst to be used in the invention is preferably a Pd catalyst. As the Pd catalyst is usable a catalyst containing Pd deposited on activated carbon or alumina. The deposition amount of Pd may be optional and commonly commercialized catalysts containing 1 to 30% by weight of Pd are preferable and catalysts containing 2 to 10% by weight of Pd are more preferable. Both dry products and hydrated products may be usable. The use amount of the catalysts is not particularly limited, however it is preferably 0.0001 times by weight, more preferably 0.001 to 0.005 times by weight on the basis of Pd weight in the raw material. If it is within the range, the reaction time is not so much prolonged and the process can be carried out economically. The Pd catalyst to be used in the process can be recovered and made reusable by solid-liquid separation on completion of the reaction.

To carry out the invention, the N-substituted nitrogenous heterocyclic compound defined by the general formula (1), water, and the catalyst are loaded into a conventional reaction apparatus and reaction is carried out under stirring condition in the co-presence of normal pressure hydrogen. That in the co-presence of normal pressure hydrogen means normal closed state of the reaction apparatus after loading hydrogen or the state of continuous ventilation of hydrogen in an open system and may include slightly pressurized state in the closed reaction apparatus produced owing to temperature increase or slightly negative pressure condition of the reaction system produced because of consumption of enclosed hydrogen by the reaction.

The reaction temperature is 0 to 100° C, preferably 10 to 90° C, and particularly preferably 20 to 80°C. Herein, in the case the N-substituted nitrogenous heterocyclic compound as a raw material is hardly water-soluble and has a melting point lower than the reaction temperature, the melted N-substituted nitrogenous heterocyclic compound probably adheres to the catalyst during the reaction to deteriorate the catalytic activity. In such a case, the reaction temperature may be lowered.

The reaction duration is generally 1 to 30 hours although it differs depending on the use amount of the catalyst and the reaction temperature.

On completion of the reaction, after the catalyst and toluene and substituted toluene are removed, the water-based solvent is concentrated and removed to precipitate an aimed nitrogenous heterocyclic compound defined by the general formula (3) or evaporated to isolate the compound.

The invention may be applicable for hydrogenolysis of a nitrogenous heterocyclic compound defined by the general formula (12): 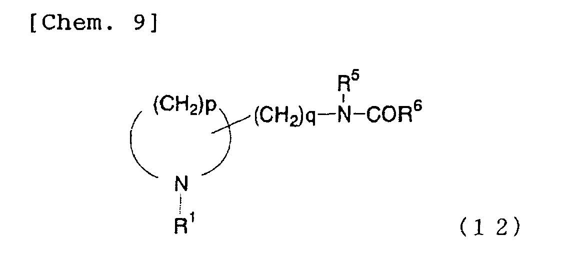 wherein R¹ denotes an (un) substituted benzyl group; R⁵ denotes a hydrogen atom or an alkyl having 1 to 4 carbon atoms; R⁶ denotes i) an alkyl having 1 to 4 carbon atoms; ii) an alkoxyl having 1 to 4 carbon atoms; iii) phenyl; iv) phenyloxy ; v) an aralkyl whose aromatic ring may be unsubstituted or substituted with an alkyl having 1 to 4 carbon atoms, an alkoxyl having 1 to 4 carbon atoms, or a halogen atom; or vi) an aralkyloxy whose aromatic ring may be unsubstituted or substituted with an alkyl having 1 to 4 carbon atoms, an alkoxyl having 1 to 4 carbon atoms, or a halogen atom; q denotes 0 or 1; and p denotes an integer of 3 to 6.

The process for producing the compound is not particularly limited and may be carried out by reaction of an N-substituted nitrogenous heterocyclic compound defined by the general formula (13): 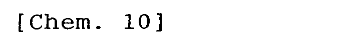 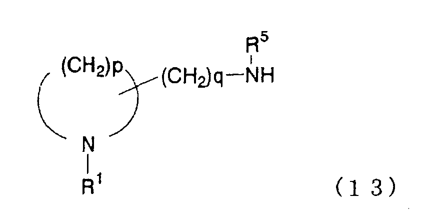 wherein R¹, R⁵, p, and q independently denote as described above with an acid halide compound or an acid anhydride to produce the compound. Herein, the acid halide compound is preferably those defined by the general formula (14) and the acid anhydride is preferably those defined by the general formula (15):

R⁶COY (14) (R⁶CO)₂O (15)

wherein R⁶ denotes as described above and Y denotes a chlorine atom or a bromine atom). The reaction is to be carried out preferably in condition of controlling pH in a range of 9 to 13 and more preferably in condition of controlling pH in a range of 9 to 13 in the co-presence of a surfactant in the water-based solvent.

The N-substituted nitrogenous heterocyclic compound defined by the general formula (12) is preferably a 1-substituted-3-aminopyrrolidine derivative defined by the general formula (16): 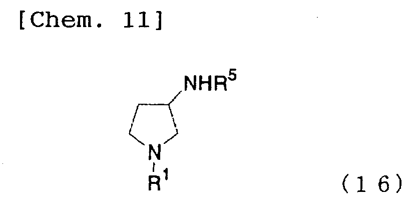 wherein R¹ and R⁵ independently denote as described above and a compound defined by the general formula (16) is reacted with an acid halide or acid anhydride to give a compound defined by the general formula (17): 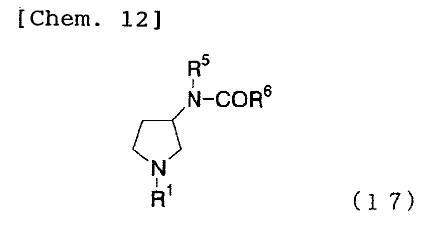 wherein R¹, R⁵, and R⁶ independently denote as described above.
Practical examples of the 1-substituted-3-aminopyrrolidine derivative defined by the general formula (16) are 1-benzyl-3-aminopyrrolidine, 1-(4-methylbenzyl)-3-aminopyrrolidine, 1-benzyl-3-methylaminopyrrolidine, 1-(4-methylbenzyl)-3-ethylaminopyrrolidine, 2-aminomethyl-1-benzylpyrrolidine, 3-amino-1-benzylpipelidine, 4-aminomethyl-1-benzylpipelidine, and 3-amino-1-benzylhexamethyleneimine. Particularly, 1-benzyl-3-aminopyrrolidine, 1-benzyl-3-methylaminopyrrolidine, and 3-amino-1-benzylpipelidine are preferable. Their optically active isomers are also usable. Practical examples may include optically active isomers with (R) structure or (S) structure at the third site such as optically active 1-substituted-3-aminopyrrolidine derivatives and optically active 1-substituted-3-aminopiperidine derivatives and also optically active 1-substituted-2-aminomethylpyrrolidine derivatives having an asymmetric center at the second site and preferable examples are optically active 1-benzyl-3-aminopyrrolidine, optically active 1-benzyl-2-methylaminopyrrolidine, and optically active 1-benzyl-3-aminopiperidine derivatives. Use of the optically active compounds of the nitrogenous heterocyclic compounds defined by the general formula (13) makes it possible to produce optically active compounds of the nitrogenous heterocyclic compounds defined by the general formulas (12) with scarcely causing racemination. The optically active compounds mean compounds containing 90% or more of either (S) isomers or (R) isomer.

The acid halide compound defined by the general formula (14) may include alkylcarboxylic acid chlorides such as acetyl chloride and butyloyl chloride; alkylcarboxylic acid bromides such as acetyl bromide and butyloyl bromide; alkyl chlorocarbonates such as ethyl chlorocarbonate and butyl chlorocarbonate; aromatic carboxylic acid chlorides such as benzoyl chloride and toluoyl chloride; aromatic carboxylic acid bromides such as benzoyl bromide and toluoyl bromide; aryl chlorocarbonate such as phenyl chlorocarbonate and tolyl chlorocarbonate; aralkylcarboxylic acid chlorides such as phenylacetyl chloride and phenylethyl chloride; and aralkyl chlorocarbonates such as benzyl chlorocarbonate and phenylethyl chlorocarbonate and preferably ethyl chlorocarbonate, butyl chlorocarbonate, benzoyl chloride, toluoyl chloride, phenyl chlorocarbonate, tolyl chlorocarbonate, phenylacetyl chloride, phenylethyl chloride, benzyl chlorocarbonate, and phenylethyl chlorocarbonate. The use amount is 0.8 to 1.5 equivalent, preferably 1.0 to 1.2 equivalent to that of the N-substituted nitrogenous heterocyclic compound defined by the general formula (13).

The acid anhydride defined by the general formula (15) may include alkylcarboxylic acid anhydrides such acetic anhydride and varelic anhydride; dialkyl dicarbonates such as dimethyl dicarbonate, diethyl dicarbonate, and di-tert-butyl dicarbonate; aromatic carboxylic acid anhydrides such as benzoic anhydride and toluic anhydride; aralkylcarboxylic acid anhydrides such as phenylacetic anhydride and phenylpropionic anhydride; and diaralkyl dicarbonates such as dibenzyl dicarbonate and preferably acetic anhydride, diethyl dicarbonate, and di-tert-butyl dicarbonate, and dibenzyl dicarbonate. The use amount is 0.8 to 1.5 equivalent, preferably 1.0 to 1.2 equivalent to that of the N-substituted nitrogenous heterocyclic compound defined by the general formula (13).

The production process is preferable to be carried out in a water-based solvent and it can be carried out without any problem if the raw materials contain a slight amount of an organic solvent. If the substrate concentration in the reaction solution is proper to make stirring possible, the reaction can be carried out, and it is generally 1 to 50% by weight, preferably 5 to 40% by weight, and particularly preferably 10 to 30% by weight. If it is within the range, the workability becomes excellent and the production yield is high.

The reaction method may be a method of dropwise adding the compound defined by the general formula (14) or (15) while the mixture of the compound defined by the general formula (13) and water is stirred. Herein, the reaction solution is adjusted to be pH in a range of 9 to 13, preferably 10 to 12, and more preferably 10 to 11. When the compound defined by the general formula (14) or (15) is dropwise added, the compound defined by the general formula (12) is produced by reaction with the compound defined by the general formula (13) and during the reaction, halogenic acid and such as hydrochloric acid and bromic acid, acetic acid, carbonic acid and the like are produced as byproducts and pH is decreased along with proceeding of the reaction and therefore, reaction is carried out while a base is dropwise added to constantly adjust pH in a range of 9 to 13. The base to be dropwise added may be tertiary amines such as trimethylamine, organic bases such as pyridine, and aqueous alkaline solutions of alkali metal hydroxides, hydrogen carbonates, and carbonates and more preferably aqueous solutions of alkali metal hydroxides such as sodium hydroxide and potassium hydroxide. The concentration of the aqueous alkaline solution may be optional, however since the production efficiency is decreased if the substrate concentration of the reaction solution is decreased, it is generally in a range of 5 to 50% and preferably 30 to 48%. The reaction temperature is -3 to 60°C and preferably 0 to 40°C. If the temperature is within the range, the compound defined by the general formula (14) or (15) can be reacted efficiently with the compound defined by the general formula (13).

The compound defined by the general formula (13) or the produced compound defined by the general formula (12) is scarcely dissolved in water, if a surfactant is added, the reaction can smoothly be promoted. The surfactant to be added for co-presence in the reaction may be commercialized quaternary ammonium salts such as tetrabutyl ammonium chloride, tri-long chain alkylmethyl ammonium chloride (C8 to C20 Arukatto 336), di-long chain alkyldimethyl ammonium chloride (C10 to C20 Cation DS), and benzalconium, and preferably tri-long chain alkylmethyl ammonium chloride (C8 to C20 Arukatto 336), di-long chain alkyldimethyl ammonium chloride (C16 to C20 Cation DS), and benzalconium, and particularly preferably di-long chain alkyldimethyl ammonium chloride (C16 to C20 Cation DS). Although it is not definitely defined and differs in accordance with the type of the surfactants and the compounds defined by the general formulas (13) and (12), the addition amount is generally 0.001 to 0.1 times by weight, preferably 0.005 to 0.05 times by weight, and more preferably 0.007 to 0.03 times by weight to the N-substituted nitrogenous heterocyclic compound defined by the general formula (13). In the case the solubility of the loaded compound defined by the general formula (13) and the produced compound defined by the general formula (12) in water is low, the reaction may not be promoted smoothly because scaling in the wall of a reaction container occurs or bulks are formed unless the surfactant is absent.

The reaction duration differs depending on the type and use amount of the substrate, and the reaction temperature, however it is generally 0.5 to 10 hours.

Consequently, the nitrogenous heterocyclic compound defined by the general formula (12) can be obtained. To isolate the compound, a method of filtering the precipitated crystal from the aqueous solution and a method of extracting the product with an organic solvent can be employed and the method of filtering the product is preferable in the case the crystal is precipitated since the environmental pollution can be suppressed to the minimum.

### Examples

Hereinafter, the invention will be more readily apparent from the following examples, however it is not intended that the invention be limited to the illustrated examples.

The composition analysis of the reaction solutions and chemical purity analysis of the distilled products in Examples were carried out by gas chromatography (GC) on the basis of area %. The GC analysis conditions cannot definitely be defined since they differ depending on the objects to be analyzed and as typical analysis conditions will be described analysis of 3-amino-1-benzylpyrrolidine (BAP) and 3-aminopyrrolidine (AP).
GC analysis conditions:
column: NEUTRA BOND-1(NB-1) <manufactured by GL Science>, I.D. 0.25 mmφ × 60 m, 0.4 µm;
column temperature: 70°C (15 min) →20°C/min→270°C (10 min); and RT: AP 11.2 min and BAP 21.1 min.
Also, the optical purity analysis differs depending on the object to be analyzed, and for example, in the case of 3-aminopyrrolidine, the measurement was carried out by causing reaction with ditoluoyl-D-tartaric acid anhydride (manufactured by Toray Industries Inc.) to produce optically active tartaric acid derivative and subjecting the derivative to a high performance liquid chromatography (HPLC) using ODS column. 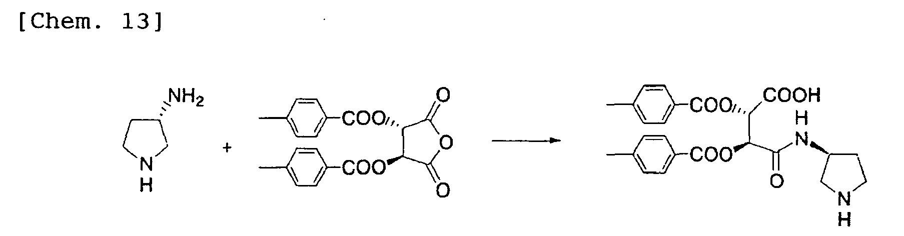 HPLC analysis conditions:
column: CAPCELL PAC C18 SG120 <manufactured by SISEIDO>; 4.6 mmφ × 150 mm;
developer: (0.03% ammonia water adjusted at pH 4.0 with acetic acid)/methanol = 50/50;
flow rate: 1.0 ml/min;
column temperature: 40°C; and
RT: R-AP derivative 24.8 min and S-AP derivative 29.1 min.

### Reference Example

### Process for producing (S)-3-amino-1-benzylpyrrolidine

Diglyme 80 g and boron sodium hydride 8.8 g (0.23 mol) were loaded to a 200 ml four-neck flask equipped with a stirrer, a dropping funnel, a Dimroth condenser, and a thermometer and while the contents being stirred under condition of ice cooling, 14.0 g (optical purity 98%/ee or higher, about 0.05 mole) of a mixture of L-aspartic acid benzylamidomethyl ester hydrochloride (referred to as ABN hydrochloride) and L-isoaspartic acid benzylamidomethyl ester hydrochloride (referred to as IABN hydrochloride) was added. Next, a solution obtained by diluting 5.7 g (0.06 mole) of concentrated sulfuric acid with diglyme 20 ml was dropwise added for about 30 minutes and the resulting mixture was stirred for 2 hours. The reaction solution was heated to 65°C and further stirred for 2 hours. On completion of the reaction, the reaction solution was vacuum-concentrated. Water 70 g was added and dissolved and then concentrated hydrochloric acid 25 g was added and the resulting reaction solution was stirred at 65°C for 4 hours. After the reaction solution was cooled to a room temperature and neutralized by adding 32 g of 46% sodium hydroxide while being stirred. Extraction was repeated 3 times with toluene 100 ml and the entire toluene layers were mixed and vacuum-concentrated. The concentrated product was vacuum distilled to obtain (S)-3-amino-1-benzylpyrrolidine 7.3 g as a fraction at 130 to 133°C/1.3 kPa. The distillate was analyzed to find it had a chemical purity of 99% and an optical purity of 96.7%ee. To improve the optical purity, a salt was produced by L-tartaric acid and recrystallized with water and after that, the salt was dissolved by sodium hydroxide and extraction with toluene and distillation were carried out to obtain (S)-3-amino-1-benzylpyrrolidine with optical purity of 99.5%ee.

### Example 1

A 500 ml four-neck flask equipped with a stirrer, a thermometer, a Dimroth condenser, and a gas introduction pipe was loaded with (S)-3-amino-1-benzylpyrrolidine 52.5 g (0.3 mole, optical purity 99.5%/ee), water 97.5 g, and 5% Pd/C 5.25 g (PE type, 55.27% water content, manufactured by N. E. Chemcat Corp.), and while the contents being stirred at 80°C, hydrogen was ventilated for 8 hours. After hydrogen ventilation was stopped, the mixture was cooled to a room temperature while being stirred and the catalyst was separated by vacuum filtration. The filtrate was vacuum concentrated to about 50 g by an evaporator. The concentrated product was distilled by a distillation apparatus equipped with about 5-step refining distillation towers packed with Heli-pack to obtain (S)-3-aminopyrraiidine 23.6 g as a fraction at 4.8 kPa. The yield was 91.0% and the chemical purity was 99.9 area% and an optical purity was 99.5%ee. The water content was 0.3%.

### Example 2

A 100 ml four-neck flask equipped with a stirrer, a thermometer, a Dimroth condenser, and a gas introduction pipe having a balloon filled with 5 1 of hydrogen at the tip end was loaded with 3-amino-1-benzylpyrrolidine 5.3 g, water 20 g, and 5% Pd/C 1.0 g (PE type, 55.27% water content, manufactured by N.E. Chemcat Corp.) and the contents were stirred at 60°C for 10 hours. When the reaction solution was analyzed by GC, and a GC chart excluding toluene showed that 3-amino-1-benzylpyrrolidine, a raw material, was completely consumed and only 3-aminopyrrolidine, a product, was detected. The yield was quantitative (about 99% or higher).

### Example 3

A 500 ml four-neck flask equipped with a stirrer, a thermometer, a Dimroth condenser, and a titration funnel was loaded with (S)-3-amino-1-benzylpyrrolidine 17.6 g (0.1 mole, optical purity 99.5%/ee), water 158.7 g, and Cation DS (manufactured by Sanyo Chemical Industries, Ltd.) 0.2 g and the pH of the mixture was adjusted to be 11±0.5 with an aqueous 48% sodium hydroxide solution. While the mixture being stirred at 50 to 60°C, di-tert-butyl dicarbonate (hereinafter abbreviated as DiBoc) 26.2 g (0.12 mole) was dropwise added for about 2 hours. During the time, the reaction solution was adjusted at pH 11±0.5 with an aqueous 48% sodium hydroxide solution. After being stirred for further 1 hour, the reaction solution was cooled to a room temperature and precipitated crystal was separated by filtration. The crystal was vacuum dried at 50°C to obtain (S)-1-benzyl-3-tert-butoxycarbonylaminopyrrolidine 26.0 g. The yield was 94.1% and the chemical purity was 99.1 % and an optical purity was 99.5%ee. The same apparatus as that of Example 1 was loaded with (S)-1-benzyl-3-tert-butoxycarbonylaminopyrrolidine 26.0 g (optical purity 99.5%ee), water 120 g, and 5% Pd/C 2.6 g (PE type, 55.27% water content, manufactured by N. E. Chemcat Corp.) and the contents were stirred at reaction temperature of 40°C for 10 hours under hydrogen ventilation. When the reaction solution was analyzed by GC, and the peak of the raw material disappeared and other than toluene only 3-tert-butoxycarbonylaminopyrrolidine was detected. After completion of the reaction, Pd/C was removed by filtration and the filtrate was concentrated to 30 g by an evaporator. Next, the concentrated product was mixed with toluene and concentrated to 20 g to remove water by azeotropic boiling. While being mixed, the concentrated solution was mixed slowly with n-hexane 25 g to precipitate a crystal and further stirred for 2 hour in an ice bath. The precipitated crystal was separated by filtration and vacuum dried to obtain (S)-3-tert-butoxycarbonylaminopyrrolidine 15.4 g. The yield was 87.4%, the chemical purity was 99.5 area%, and an optical purity was 99.5 %ee. The water content was 0.4%.

### Example 4

Reaction was carried out for 10 hours under stirring condition in the same manner as Example 2, except that 3-amino-1-p-toluylpyrrolidine 5.8 g was used in place of 3-amino-1-benzylpyrrolidine 5.3 g and the reaction temperature was lowered to 60°C. When the reaction solution was analyzed by GC, the peak of the raw material disappeared and 3-aminopyrrolidine was produced.

### Example 5

A 200 ml four-neck flask equipped with a stirrer, a thermometer, a Dimroth condenser, and a dropping funnel was loaded with 3-amino-1-benzylpyrrolidine 3.5 g (0.02 mole), water 100 g, and Cation DS (manufactured by Sanyo Chemical Industries, Ltd.) 0.1 g and the pH of the mixture was adjusted to be 11±0.5 with an aqueous 48% sodium hydroxide solution. While the mixture being stirred at 30 to 40°C, benzyl chlorocarbonate 4.1 g (0.024 mole) was dropwise added for about 1 hour. During the time, the reaction solution was adjusted at pH 11±0.5 with an aqueous 48% sodium hydroxide solution. After being stirred for further 1 hour, the reaction solution was cooled to a room temperature and precipitated crystal was separated by filtration. The crystal was vacuum dried at 50°C to obtain 1-benzyl-3-benzyloxycarbonylaminopyrrolidine 5.7 g. The yield was 91.0% and the chemical purity was 98.6%.

A 100 ml four-neck flask equipped with a stirrer, a thermometer, a Dimroth condenser, and a gas introduction pipe was loaded with 1-benzyl-3-benzyloxycarbonylaminopyrrolidine 5.7 g, water 20 g, methanol 1 g, and 5% Pd/C 1.0 g (PE type, 55.27% water content, manufactured by N.E. Chemcat Corp.) and the mixture was stirred at 50°C for 20 hours under hydrogen ventilation. When the reaction solution was analyzed by GC, and according to a GC chart excluding toluene, 1-benzyl-3-benzyloxycarbonylaminopyrrolidine, a raw material, was 5.2 area% and 3-benzyloxycarbonylaminopyrrolidine, a product, was 92.8 area%.

### Example 6

Reaction was carried out at 60°C for 10 hours under stirring condition in the same manner as Example 2, except that methanol 20 g was used in place of water. When the reaction solution was analyzed by GC, according to a GC chart excluding toluene, 3-amino-1-benzylpyrrolidine, a raw material, was 83 area% and 3-aminopyrrolidine, a product, was 17 area%.

### Example 7

Reaction was carried out at 80°C for 10 hours under stirring condition in the same manner as Example 2, except that propanol 20 g was used in place of water. When the reaction solution was analyzed by GC, 3-aminopyrrolidine, a product, was slightly produced and 3-amino-1-benzylpyrrolidine, a raw material, was left.

### Example 8

Reaction was carried out at 60°C for 10 hours under stirring condition in the same manner as Example 2, except that methanol 20 g was used in place of water and the Pd catalyst to be added was increased as much as 2 times. When the reaction solution was analyzed by GC, according to a GC chart excluding toluene, 3-amino-1-benzylpyrrolidine, a raw material, was 75 area% and 3-aminopyrrolidine, a product, was 25 area%.

### Example 9

A 100 ml four-neck flask equipped with a stirrer, a thermometer, a Dimroth condenser, and a gas introduction pipe was loaded with 1-benzyl-3-methylpiperazine 9.5 g (50 mmole), water 50 g, and 5% Pd/C 1.0 g (PE type, 55.27% water content, manufactured by N.E. Chemcat Corp.) and the contents were stirred at 40°C for 6 hours under hydrogen ventilation condition. When the reaction solution was analyzed by GC, a GC chart excluding toluene showed that no 1-benzyl-3-methylpiperazine, a raw material, was detected and only a peak of 2-methylpiperazine was detected. The yield was quantitative (about 99% or higher).

### Example 10

The same apparatus as that used in Example 9 was loaded with 3-amino-1-benzyl-4-hydroxypyrrolidine 9.6 g and reacted for 10 hours in the same manner, and a GC chart excluding toluene showed that no raw material was detected and only a peak of 3-amino-4-hydroxypyrrolidine was detected. The yield was quantitative (about 99% or higher).

### Example 11

The same apparatus as that used in Example 9 was loaded with 3-benzylamino-1-benzyl-4-hydroxypyrrolidine 9.6 g and reacted for 15 hours in the same manner, and a GC chart excluding toluene showed that no raw material was detected and only a peak of 3-amino-4-hydroxypyrrolidine was detected. The yield was quantitative (about 99% or higher).

### Example 12

A 500 ml four-neck flask equipped with a stirrer, a thermometer, a Dimroth condenser, and a gas introduction pipe was loaded with (S)-1-benzyl-3-hydroxypyrrolidine 52.5 g (0.3 mole, optical purity 99.5%ee), water 210 g, and 5% Pd/C 5.25 g (PE type, 55.27% water content, manufactured by N.E. Chemcat Corp.) and the contents were stirred at 80°C for 8 hours under hydrogen ventilation condition. The hydrogen ventilation was stopped and the resulting reaction solution was cooled to a room temperature while being stirred and the catalyst was removed by vacuum filtration. The filtrate was concentrated to about 50 g by an evaporator. The concentrated product was distilled by a distillation apparatus equipped with about 5-step refining distillation towers packed with Heli-pack to obtain (S)-3-hydroxypyrrolidine 23.5 g as a fraction at 1 kPa and tower summit temperature of 93 to 95°C. The yield was 90% and the chemical purity was 99.9 area% and an optical purity was 99 . 5 %ee. The water content was 0.3%.

### Industrial Applicability

The invention provides a method of de-protection of a nitrogenous heterocyclic compound protected with N-benzyl group by employing a conventionally widely used facility by normal pressure hydrogen and the invention is' applicable for debenzylation of amino group in a side chain.

## Claims

1. A process for producing a nitrogenous heterocyclic compound defined by the general formula (3): 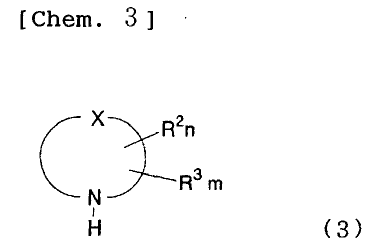 wherein R² and R³ independently denote i) a hydrogen atom; ii) a lower alkyl having 1 to 4 carbon atoms; iii) a lower alkoxyl having 1 to 4 carbon atoms; iv) a hydroxyl group; v) a mercapto group; vi) an (un)substituted amino group; vii) an aryl whose aromatic ring may be unsubstituted or substituted with an alkyl having 1 to 4 carbon atoms, an alkoxyl having 1 to 4 carbon atoms, or a halogen atom; or viii) an aralkyl whose aromatic ring may be unsubstituted or substituted with an alkyl having 1 to 4 carbon atoms, an alkoxyl having 1 to 4 carbon atoms, or a halogen atom and may be the same or different groups; m and n independently denote an integer of 0 to 3; and X denotes a residual group of a nitrogenous heterocyclic ring defined by the general formula (2): 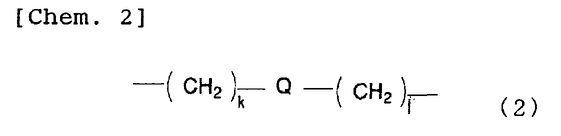 wherein Q denotes CH₂, NR⁴, or O, wherein R⁴ denotes i) a hydrogen atom; ii) a lower alkyl having 1 to 4 carbon atoms; iii) a lower alkoxyl having 1 to 4 carbon atoms; iv) an aryl whose aromatic ring may be unsubstituted or substituted with an alkyl having 1 to 4 carbon atoms, an alkoxyl having 1 to 4 carbon atoms, or a halogen atom; v) an aralkyl whose aromatic ring may be unsubstituted or substituted with an alkyl having 1 to 4 carbon atoms, an alkoxyl having 1 to 4 carbon atoms, or a halogen atom; or vi) an aralkyloxy whose aromatic ring may be unsubstituted or substituted with an alkyl having 1 to 4 carbon atoms, an alkoxyl having 1 to 4 carbon atoms, or a halogen atom; k and l independently denote an integer of 1 to 4; and k + l is 3 to 6: by carrying out hydrogenolysis of an N-substituted nitrogenous heterocyclic compound defined by the general formula (1): 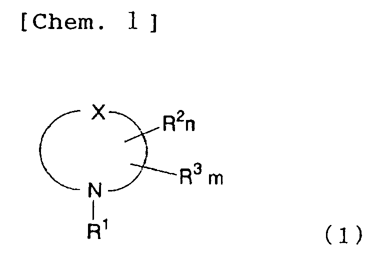 wherein R¹ denotes an (un)substituted benzyl group; R², R³, X, m, and n are the same as described: with normal pressure hydrogen in the presence of a catalyst.

2. The process for producing the nitrogenous heterocyclic compound according to claim 1, wherein the catalyst is Pd.

3. The process for producing the nitrogenous heterocyclic compound according to claim 1 or 2, wherein the hydrogenolysis is carried out in a water-based solvent.

4. The process for producing the nitrogenous heterocyclic compound according to any one of claims 1 to 3, wherein the N-substituted nitrogenous heterocyclic compound defined by the general formula (1) is one of a N-substituted nitrogenous heterocyclic compound defined by the following general formulas (4) to (7): 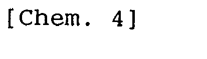 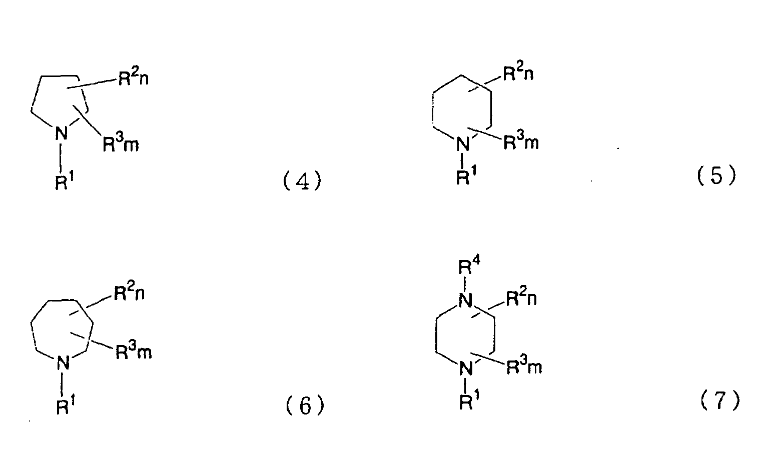 wherein R¹, R², R³, R⁴ , m, and n are the same as described: and the nitrogenous heterocyclic compound defined by the general formula (3) is a nitrogenous heterocyclic compound defined by the following general formulas (8) to (11): 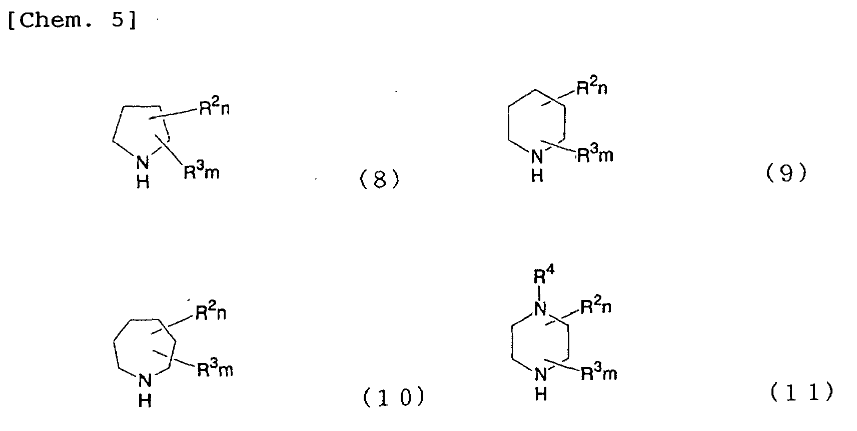 wherein R², R³, R⁴, m, and n are the same as described.

5. The process for producing the nitrogenous heterocyclic compound according to claim 4, wherein R², R³, and R⁴ independently denote a group selected from a hydrogen atom, methyl, aminomethyl, hydroxymethyl, ethyl, hydroxyl, amino, methylamino, benzylamino, ethoxycarbonylamino, tert-butoxycarbonylamino, benzyloxycarbonylamino, methoxy, and benzyl in the general formulas (4) to (11).

6. The process for producing the nitrogenous heterocyclic compound according to claim 4, wherein the N-substituted nitrogenous heterocyclic compound defined by the general formulas (4-) to (7) is a compound selected from 3-amino-1-benzylpyrrolidine, 3-amino-1-(4-methylbenzyl)pyrrolidine, 3-methylamino-1-benzylpyrrolidine, 1-benzyl-3-tert-butoxycarbonylaminopyrrolidine, 3-benzylamino-1-benzylpyrrolidine, 1-benzyl-3-hydroxypyrrolidine, 1-benzyl-3-methoxypyrrolidine, 3-amino-1-benzyl-4-hydroxypyrrolidine, 3-benzylamino-1-benzyl-4-hydroxypyrrolidine, 2-aminomethyl-1-benzylpyrrolidine, 2-hydroxymethyl-1-benzylpyrrolidine, 3-ethoxycarbonylamino-1-(4-methylbenzyl)pyrrolidine, 1-benzyl-3-benzyloxycarbonylaminopyrrolidine, 3-amino-1-benzylpiperidine, 1-benzyl-3-methylpiperidine, 3-amino-1-benzylhexamethyleneimine, 1-benzyl-3-methylpiperazine, and 1,4-dibenzyl-3-methylpiperazine.

7. The process for producing the nitrogenous heterocyclic compound according to any one of claims 1 to 6, wherein the N-substituted nitrogenous heterocyclic compound defined by any one of the general formulas (1) and (4) to (7) is an optically active compound and the nitrogenous heterocyclic compound defined by any one of the general formulas (3) and (8) to (11) is an optically active compound.

8. The process for producing the nitrogenous heterocyclic compound according to any one of claims 1 to 3, wherein the N-substituted nitrogenous heterocyclic compound defined by the general formula (1) is a compound defined by the general formula (12): 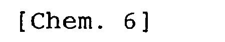 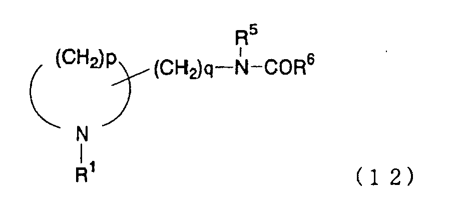 wherein R¹ denotes an (un)substituted benzyl group; R⁵ denotes a hydrogen atom or an alkyl having 1 to 4 carbon atoms; R⁶ denotes i) an alkyl having 1 to 4 carbon atoms; ii) an alkoxyl having 1 to 4 carbon atoms; iii) phenyl; iv) phenyloxy; v) an aralkyl whose aromatic ring may be unsubstituted or substituted with an alkyl having 1 to 4 carbon atoms, an alkoxyl having 1 to 4 carbon atoms, or a halogen atom; or vi) an aralkyloxy whose aromatic ring may be unsubstituted or substituted with an alkyl having 1 to 4 carbon atoms, an alkoxyl having 1 to 4 carbon atoms, or a halogen atom; q denotes 0 or 1; and p denotes an integer of 3 to 6.

9. The process for producing the nitrogenous heterocyclic compound according to claim 8, wherein the N-substituted nitrogenous heterocyclic compound defined by the general formula (12) is a compound obtained by reaction of an N-substituted nitrogenous heterocyclic compound defined by the general formula (13): 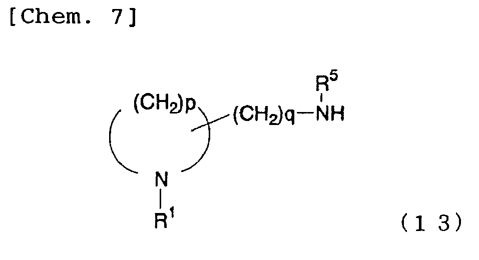 wherein R¹, R⁵, p, and q independently denote as described above with an acid halide compound or an acid anhydride.

10. The process for producing the nitrogenous heterocyclic compound according to claim 9, wherein the acid halide compound or the acid anhydride is defined by the general formula (14) or (15):
R⁶COY (14) (R⁶CO)₂O (15)
wherein R⁶ denotes as described above and Y denotes a chlorine atom or a bromine atom.

11. The process for producing the nitrogenous heterocyclic compound according to claim 9 or 10, wherein the reaction is carried out while pH is controlled to be in a range of 9 to 13.

12. The process for producing the nitrogenous heterocyclic compound according to any one of claims 9 to 11, wherein the reaction is carried out in a water-based solvent.

13. The process for producing the nitrogenous heterocyclic compound according to any one of claims 9 to 12, wherein the reaction is carried out in co-presence of a surfactant.

14. The process for producing the nitrogenous heterocyclic compound according to any one of claims 9 to 13, wherein the acid anhydride defined by the general formula (15) is di-tert-butyl dicarbonate.

15. The process for producing the nitrogenous heterocyclic compound according to claim 13 or 14, wherein the surfactant is an alkyl ether sulfonic acid salt or a quaternary ammonium salt.

16. The process for producing the nitrogenous heterocyclic compound according to any one of claims 8 to 15, wherein the N-substituted nitrogenous heterocyclic compound defined by the general formula (13) is an optically active compound and the nitrogenous heterocyclic compound defined by the general formula (12) is also an optically active compound.

17. The process for producing the nitrogenous heterocyclic compound according to any one of claims 8 to 16, wherein the 1-substituted nitrogenous heterocyclic compound defined by the general formula (13) is a 1-substituted-3-aminopyrrolidine derivative defined by a general formula (16): 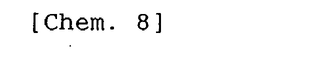 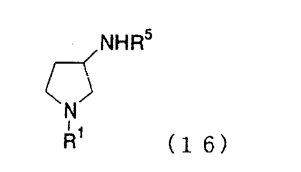 wherein R¹ and R⁵ independently denote as described above and the nitrogenous heterocyclic compound defined by the general formula (12) is a 3-substituted-aminopyrrolidine compound defined by the general formula (17): 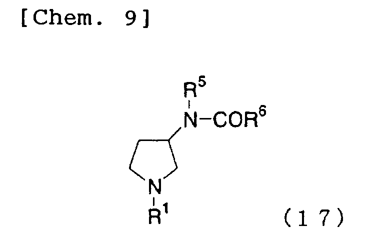 wherein R¹, R⁵, and R⁶ independently denote as described above.
